# FASCICULE DE BREVET EUROPEEN

(11) **EP 0 662 813 B1**
(45) Date de publication et mention de la délivrance du brevet: **14.10.1998**
(21) Numéro de dépôt: 93917837.2
(22) Date de dépôt: 29.07.1993
(51) Int. Cl.: A61K 7/032, A61K 7/48

(54) **COMPOSITION COSMETIQUE CONTENANT DE L'ACIDE DESOXYRIBONUCLEIQUE ET UNE CIRE**
Kosmetische Zusammensetzzungen enthaltend eine Desoxyribonukleinsäureund eine Waxe
COSMETIC COMPOSITION CONTAINING DEOXYRIBONUCLEIC ACID AND A WAX

(43) Date de publication de la demande: 19.07.1995
(73) Titulaire: L'OREAL, 75008 Paris (FR)
(72) Inventeur: PIOT, Bertrand, F-92250 La Garenne-Colombe (FR); PATRAUD, Jeanne, F-75013 Paris (FR); FELARDOS, Christian, F-94450 Chevilly Larue (FR)
(74) Mandataire: Dodin, Catherine
(86) Numéro de dépôt international: FR9300776
(87) Numéro de publication internationale: WO9503774

(56) Documents cités:
- EP-A- 0 106 762
- EP-A- 0 378 519
- EP-A- 0 416 677
- CH-A- 678 489
- FR-A- 2 205 304
- FR-A- 2 511 243
- US-A- 5 116 607

## Description

La présente invention concerne l'utilisation d'une composition contenant des cires pour le maquillage des yeux ainsi qu'un procédé de maquillage des yeux utilisant une telle composition.

Il est connu d'utiliser des cires dans des compositions pour le maquillage des yeux. Mais, ces compositions essentiellement à base de cire donnent un maquillage de qualité médiocre.

On connait également l'ajout d'un agent filmogène, de type polymère, en solution dans la phase aqueuse de la composition cosmétique. Mais cet ajout n'est pas toujours satisfaisant au niveau du résultat procuré après application sur les cils.

On connait également l'incorporation d'acide désoxyribonucléique dans des compositions cosmétiques dans le but de favoriser la regénération cellulaire au niveau de la peau ou pour la repousse des cheveux, ou de stimuler les activités anti-séborrhéiques.

Des compositions cosmétiques à base d'acide désoxyribonucléique et de cires pour l'application sur la peau sont aussi connues dans l'état de la technique, et notamment dans les documents EP-A-416677, CH-B-678489, FR-A-2205304, FR-A-2511243, ainsi que dans le brevet USA-5116607 pour l'application capillaire.

Mais l'acide désoxyribonucléique n'est pas connu comme composant entrant dans des compositions pour le maquillage des cils et des sourcils.

Le procédé de maquillage selon l'invention a permis de pallier les problèmes mentionnés précédemment. Notamment, il a été découvert de façon surprenante que l'ajout d'acide désoxyribonucléique dans une composition pour le maquillage des yeux améliore de manière substantielle les qualités cosmétiques telles que l'allongement et le recourbement des cils.

La présente invention a pour objet un procédé de maquillage des yeux caractérisé par le fait que l'on applique sur les cils et/ou les sourcils une composition comprenant, éventuellement en présence d'un support cosmétiquement acceptable, de l'acide désoxyribonucléique ou son sel minéral ou organique et au moins une cire.

Le rapport en poids de la quantité d'acide désoxyribonucléique par rapport à la quantité de cire peut être compris entre 0,025 et 2,5.

Le poids moléculaire de l'acide désoxyribonucléique peut être compris entre 1.10⁵ et 1.10⁷, de préférence entre 5.10⁵ et 5.10⁶.

La quantité d'acide désoxyribonucléique peut être comprise entre 0,02 % et 5 % en poids en matière sèche de polymère, de préférence comprise entre 0,05 et 2 % en poids de matière sèche de polymère par rapport à l'ensemble des polymères qui peuvent être présents dans la composition.

Les cires utilisées dans la composition de maquillage peuvent être choisies parmi des cires animales, végétales, minérales, synthétiques ou leur mélange.

La composition pour le procédé de maquillage des yeux peut comprendre également des pigments choisis parmi des pigments minéraux, organiques éventuellement nacrés. Ces pigments sont compris en une proportion allant de 3 à 25 % en poids par rapport au poids total de la composition suivant la coloration et l'intensité de la coloration que l'on cherche à obtenir.

La composition utilisée pour le procédé selon la présente invention peut se présenter sous forme d'émulsions telles que huile-dans-eau, ou eau-dans-huile, sous forme de suspension en milieu solvant, sous forme de microdispersions ou encore sous forme solide ou pâteuse anhydre.

Lorsqu'elle est utilisée sous forme d'émulsion, la composition peut contenir des agents tensio-actifs présents en une proportion comprise entre 2 et 30 % en poids par rapport au poids total de la composition. Ces agents tensio-actifs sont choisis parmi des agents tensio-actifs anioniques ou non ioniques.

Le procédé de préparation de la composition de maquillage consiste à mélanger de l'acide désoxyribunocléique à une phase huileuse contenant au moins une cire et éventuellement des agents tensio-actifs.

Selon ce procédé, l'acide désoxyribunocléique peut être présent dans une phase aqueuse. Un pigment peut être ajouté dans la phase huileuse.

Les composants des phases huileuse et aqueuse peuvent être indépendamment dissous ou fondus à une température de 85 °C puis mélangés.

La présente invention va être décrite de façon plus détaillée.

L'acide désoxyribonucléique utilisé selon l'invention peut être d'origine soit animale soit végétale et, peut se présenter sous la forme d'un de sels minéraux ou organiques. L'acide désoxyribonucléique déjà utilisé dans le domaine de l'ophtalmologie est appréciable pour l'utilisation d'une composition pour le maquillage des yeux telle que le mascara, car cet acide est très bien toléré par l'oeil.

Les cires choisies selon l'invention possèdent en règle générale un point de fusion compris entre 60 et 110 °C et ont une pénétration à l'aiguille, à 25 °C, comprise entre 3 et 40, telle que mesurée selon la norme américaine ASTM D 5 ou selon la norme française NFT 004. Le principe de la mesure de pénétration d'une aiguille selon les normes ASTM D 5 et NFT 004 consistent à mesurer la profondeur exprimée en dixième de millimètres, à laquelle pénètre une aiguille normalisée qui pèse 2,5 g placée dans un porte-aiguilles pesant 47,5 g soit un total de 50 g, l'aiguille étant placée sur la cire pendant 5 secondes.

Les cires utilisables dans la présente invention peuvent être choisies parmi les cires animales, les cires végétales, les cires minérales, les cires synthétiques et les fractions diverses de cires naturelles, toutes les cires présentant les deux caractéristiques physiques mentionnées précédemment.

Parmi les cires animales, on peut citer les cires d'abeille, les cires de lanoline et les cires d'insectes de Chine.

Parmi les cires végétales, on peut citer les cires de riz, les cires de Carnauba, de Candelila, d'Ouricurry, les cires de fibres de liège, les cires de canne à sucre, les cires du Japon, la cire de Sumac, la cire de coton.

Parmi les cires minérales, on peut citer les paraffines, les cires micro-cristallines, les cires de Montan et les ozokérites.

Parmi les cires synthétiques, on peut utiliser notamment les cires de polyéthylène, les cires obtenues par la synthèse de Fischer et Tropsch, les copolymères cireux ainsi que leurs esters.

Il est également possible d'utiliser des huiles animales ou végétales hydrogénées qui répondent toujours aux deux caractéristiques physiques mentionnées précédemment. Parmi ces huiles, on peut citer les cires de jojoba hydrogénées et les huiles hydrogénées qui sont obtenues par hydrogénation catalytique de corps gras composes de chaîne grasse linéaire ou non en C₈-C₃₂, l'huile de tournesol hydrogénée, l'huile de ricin hydrogénée, l'huile de coprah hydrogénée et la lanoline hydrogénée.

Les cires utilisables selon la présente invention sont de préférence solides et rigides à température inférieure à 50 °C.

Ces cires peuvent se présenter sous forme de dispersions stables de particules colloïdales de cire telles qu'elles peuvent être préparées selon des méthodes connues, telles que "Microémulsions Theory and Practice", L. M. Prince Ed., Academic Pross (1977), pages 21-32.

Outre, les cires et l'acide désoxyribonucléique, la composition pour le procédé selon l'invention peut comprendre également des pigments choisis parmi des pigments minéraux, des pigments organiques et des pigments nacrés. Parmi des pigments minéraux, il est possible d'utiliser le dioxyde de titane (rutile ou anatase) éventuellement traité en surface et codifié dans le Color Index sous la référence CI 77891, les oxydes de fer noir, jaune, rouge, de références respectives CI 77499, CI 77492, CI 77491, le violet de manganèse de référence CI 77742, le bleu outremer de référence CI 77007, l'oxyde de chrome de référence CI 77288, l'hydrate de chrome de référence CI 77289 et le bleu ferrique de référence CI 77510.

Les pigments organiques peuvent être choisis parmi le noir de carbone, les pigments D et C red n° 19 de référence CI 45170, D et C red n° 9 de référence CI 15585, D et C red n° 21 de référence CI 45380, D et C orange n° 4 de référence CI 15 510, D et C orange n° 5 de référence CI 45 370, D et C red n° 28 de référence CI 45 410, D et C red n° 13 de référence CI 15 630, D et C red n° 57 de référence CI 15 850, D et C yellow n° 23 de référence CI 19 140, D et C red n° 36 de référence CI 12 085, D et C Acid red n° 95 de référence CI 45 425, D et C yellow n° 6 de référence CI 15 985, D et C red n° 30 de référence CI 73 360, D et C red n° 3 de référence CI 45 430, et les laques à base de carmin de cochenille de référence CI 75 470.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition pour le procédé selon l'invention pouvant se présenter sous forme d'émulsions comprendra également des agents tensio-actifs choisis parmi des agents tensio-actifs anioniques ou non ioniques.

Parmi les agents tensio-actifs anioniques qui peuvent être utilisés seuls ou en mélange, on peut citer en particulier les sels alcalins, les sels d'ammonium, les sels d'amine ou les sels d'amino-alcool des composés suivants :
- les alcoysulfates, alcoyléther sulfates, alcoylamides sulfates et éthers sulfates, alcoylarylpolyéthersulfates, monoglycérides sulfates,
- les alcoylsulfonates, alcoyl amides sulfonates, alcoylarylsulfonates, -oléfines sulfonates, paraffines sulfonates,
- les alcoylsulfosuccinates, alcoyléthersulfosuccinates, alcoylamides sulfosuccinates,
- les alcoylsulfosuccinamates,
- les alcoylsulfoacétates, les alcoylpolyglycérol carboxylates,
- les alcoylphosphates/alcoylétherphosphates,
- les alcoylsarcosinates, alcoylpolypeptidates, alcoylamidopolypeptidates, alcoyliséthionates, alcoyltaurates.

Le radical alcoyle dans tous ces composés désigne généralement une chaîne carbonée comprenant de 12 à 18 atomes de carbone.

Parmi les agents tensio-actifs anioniques, il est possible d'utiliser les sels d'acide gras tel que l'acide oléique, l'acide ricinoléique, l'acide palmitique, l'acide stéarique, les acides d'huile de coprah ou d'huile de coprah hydrogénée et, les sels d'amine tels que les stéarate d'amine.

On peut également citer les acyl lactylates dont le radical acyl comprend de 8 à 20 atomes de carbone, et les acides carboxyliques d'éthers polyglycoliques répondant à la formule :

Alk- ( OCH₂ - CH₂)n - OCH₂ - COOH

sous forme acide ou salifiée dans lequel le substituant Alk correspond à une chaine carbonée linéaire ayant de 12 à 18 atomes de carbone et où n est une valeur statistique entière comprise entre 5 et 15.

Parmi les agents tensio-actifs non ioniques qui peuvent être utilisés seuls ou en mélange, on peut citer notamment les alcools, les alcoylphénols et les acides gras polyéthoxylés, polypropoxylés ou polyglycérolés à chaîne grasse comportant de 8 à 18 atomes de carbone.

On peut également citer des copolymères d'oxyde d'éthylène et de propylène, des condensats d'oxyde d'ethylène et de propylène sur des alcools gras, des amides gras polyéthoxylés, des amines grasses polyéthoxylées, des éthanolamides, des esters d'acide gras de glycol, des esters d'acide gras de sorbitan oxyéthylénés ou non, des esters d'acide gras du saccharose, des esters d'acide gras des polyéthylénéglycols, des triésters phosphoriques, des esters d'acide gras de dérivés du glucose. D'autres composés qui peuvent entrer également dans cette classe sont les suivants :
- les produits de condensation d'un monoalcool, d'un alpha-diol, d'un alcoylphénol, d'un amide ou d'un diglycolamide avec le glycidol ou un précurseur de glycidol tel que ceux de formule :

   R₄ - CHOH - CH₂ - O - (CH₂ - CHOH - CH₂ - O)ₚ H

   dans laquelle R₄ désigne un radical aliphatique, cycloaliphatique ou arylaliphatique, ayant de préférence entre 7 et 21 atomes de carbone et leurs mélanges, les chaînes aliphatiques pouvant comporter des groupements éther, thioéther ou hydroxyméthylène et où p qui représente une valeur statistique entière est compris entre 1 et 10 inclus, tel que décrit dans le brevet français FR 2 091 516.
- les composants répondant à la formule :

   R₅O - [C₂H₃O - (CH₂OH)]_{q} H

   dans laquelle R₅ désigne un radical alcoyle, alcényle ou alcoylaryle et où q qui est une valeur statistique entière est compris entre 1 et 10 inclus tels que décrits dans le brevet FR 1 477 048.
- les composés répondant à la formule :

   R₆ - CONH - CH₂ - CH₂O - CH₂ - CH₂ - O - (CH₂ - CHOH - CH₂ - O)ᵣ H

   dans laquelle R₆ désigne un radical ou un mélange de radicaux aliphatiques linéaires ou ramifiés, saturés ou insaturés pouvant comporter éventuellement un ou plusieurs groupement(s) hydroxyle et ayant entre 8 et 30 atomes de carbone. Ces composés peuvent être d'origine naturelle ou synthétique et r représente une valeur statistique entière comprise entre 1 et 5 et désigne le degré de condensation moyen, tels que décrits dans le brevet français FR 2 328 763.

La composition pour le procédé selon l'invention sous forme d'émulsion non ionique est constituée principalement d'un mélange d'huiles et/ou d'alcool gras, ou bien d'alcools polyéthoxylés ou polyglycérolés tels que des alcools stéarylique ou cétylstéarylique polyéthoxylés.

Les compositions pour le procédé selon l'invention sous forme d'émulsion anionique sont constituées préférentiellement à partir de stéarates d'amines.

La composition pour le procédé selon l'invention peut également contenir des ingrédients utilisés en cosmétique et choisis parmi les vitamines, les oligoléléments, les adoucissants, les conservateurs, les séquestrants, les parfums, les épaississants, les huiles, les silicones, les agents de cohésion, les polymères ainsi que les agents alcalinisants ou acidifiants habituellement utilisés dans le domaine cosmétique, des charges.

Parmi les charges, on peut utiliser notamment :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

Les épaississants entrant dans la composition pour le procédé selon l'invention peuvent être choisis parmi les épaississants naturels ou synthétiques. Parmi les épaississants naturels, on peut citer les gommes de diverses sortes telles que les gommes arabique, de guar ou de caroube.

Parmi les épaississants de synthèse, on peut citer les dérivés cellulosiques comme l'hydroxyéthylcellulose, la carboxyméthylcellulose, les dérivés de l'amidon, les dérivés d'éthers de cellulose possédant des groupes ammonium quaternaire, les polysaccharides cationiques, les sels de polymères acryliques ou méthacryliques, les polyènes ou les polysiloxanes.

Il est également possible d'obtenir un épaississement de la composition par un mélange de polyéthylène glycol et de stéarate et/ou de distéarate de polyéthylèneglycol ou par un mélange d'esters phosphoriques et d'amides gras.

Des exemples concrets mais nullement limitatifs illustrant l'invention vont maintenant être donnés.

### MODE OPERATOIRE DES EXEMPLES 1 ET 2

Les composants de la phase A sont amenés à une température d'environ 85 °C, on y ajoute les composants la phase B et on mélange le tout à la turbine. On fait bouillir l'eau de la préparation et on y dissout les conservateurs. A une température d'environ 85 °, les composants de la phase C sont ajoutés à la phase aqueuse. Les composants de la phase A, à une température de 80 °C, sont ajoutés à la phase aqueuse, mélangés à la la turbine à une température d'environ 30 °C pour donner une émulsification. Les composants de la phase D sont éventuellement ajoutés et mélangés à la pâle.

### MODE OPERATOIRE DE L'EXEMPLE 3

Les composants de la phase A sont fondus, on y ajoute les pigments et on mélange. Les composants de la phase B sont mélangés et sont ajoutés aux composants de la phase A. L'acide désoxyribonucléique est ajouté à ce mélange.

### EXEMPLES

| EXEMPLE N° 1 : EMULSION HUILE-DANS-EAU | |
|---|---|
| **A** | |
| Stéarate de triéthanolamine | 8 % |
| Stéarate de glycérol (vendu sous la dénomination "GELEOL" par la Société GATTEFOSSE) | 2 % |
| Cire d'abeille | 7 % |
| Cire de Carnauba | 2 % |
| Paraffine | 3,5 % |
| **B -** Oxyde de fer noir | 6 % |

| **C -** | |
|---|---|
| Hyroxyéthylcellulose (vendu sous la dénomination "CELLOSIZE QP" par la Société AMERCHOL) | 0,5 % |
| Gomme arabique | 2 % |
| Hydrolysat de kératine (vendu sous la dénomination "KERASOL" par la Société CRODA) | 1,8 % |
| Acide désoxyribonucléique (extrait de poisson) (vendu par la Société JAVENECH) | 0,05 % |
| **D -** Panthénol | 1,0 % |
| Conservateur | qsp |
| Eau qsp | 100 % |

| EXEMPLE COMPARATIF 1' : EMULSION HUILE-DANS-EAU | |
|---|---|
| **A** | |
| Stéarate de triéthanolamine | 8 % |
| Stéarate de glycérol (vendu sous la dénomination "GELEOL" par la Société GATTEFOSSE) | 2 % |
| Cire d'abeille | 7 % |
| Cire de Carnauba | 2 % |
| Paraffine | 3,5 % |
| **B -** Oxyde de fer noir | 6 % |

| **C -** | |
|---|---|
| Hyroxyéthylcellulose (vendu sous la dénomination "CELLOSIZE QP" par la Société AMERCHOL) | 0,5 % |
| Gomme arabique | 2 % |
| Hydrolysat de kératine (vendu sous la dénomination "KERASOL" par la Société CRODA) | 1,8 % |
| **D -** Panthénol | 1,0 % |
| Conservateur | qsp |
| Eau qsp | 100 % |

A l'inverse de l'exemple 1, cet exemple ne comprend pas d'acide désoxyribonucléique. Un test comparatif a été établi en aveugle sur un panel constitué de 19 femmes entre les exemples 1 et 1'. Le test de STUDENT a montré que l'exemple 1 comprenant l'acide désoxyribonucléique permet d'obtenir une meilleure séparation et un meilleur allongement des cils après application.

| EXEMPLE N° 2 : EMULSION HUILE-DANS-EAU | |
|---|---|
| **A -** | |
| Stéarate de triéthanolamine | 10 % |
| Cire d'abeille | 8 % |
| Cire de Carnauba | 2 % |
| Paraffine | 5 % |
| **B -** Oxyde de fer noir | 5 % |

| **C -** | |
|---|---|
| Hydroxyéthylcellulose (vendu sous la dénomination "CELLOSIZE QP" par la Société AMERCHOL) | 1,2 % |
| Acide désoxyribonucléique (extrait de germe de blé) (vendu par la Société INOCOSM) | 2 % |
| Conservateur | qsp |
| Eau qsp | 100 % |

| EXEMPLE N° 3 : DISPERSION | |
|---|---|
| **A -** | |
| Cire de paraffine | 4 % |
| Cire de Carnauba | 3 % |
| Cire d'abeille | 5 % |
| Cire de Candellila | 5 % |
| Oxyde de fer noir | 5 % |

| **B -** | |
|---|---|
| Montmorillonite | 5 % |
| Propylène carbonate | 1 % |
| Isoparaffine | 55,3 % |

| **C -** | |
|---|---|
| Acide désoxyribonucléique dans eau à 3 % de matière active (protéine de poisson) (vendu par la Société SEDERMA) | 16,7 % |

## Revendications

1. Procédé de maquillage des yeux, caractérisé par le fait que l'on applique sur les cils et/ou les sourcils, une composition comprenant de l'acide désoxyribonucléique ou l'un de ses sels minéraux ou organiques et au moins une cire.

2. Procédé selon la revendication 1, caractérisé en ce que la composition comprend un support cosmétiquement acceptable.

3. Procédé sur l'une des revendications 1 ou 2, caractérisé en ce que le rapport en poids de la quantité d'acide désoxyribonucléique par rapport à la quantité de cire est compris entre 0,025 et 2,5.

4. Procédé sur l'une des revendications précédentes, caractérisé en ce que le poids moléculaire de l'acide désoxyribonucléique est compris entre 1.10⁵ et 1.10⁷.

5. Procédé sur la revendication 5, caractérisé en ce que le poids moléculaire de l'acide désoxyribonucléique est compris entre 5.10⁵ et 5.10⁶.

6. Procédé selon l'une des revendications précédentes, caractérisé en ce que la quantité d'acide désoxyribonucléique est comprise, dans la composition, entre 0,02 % et 5 % en poids de matière sèche de polymère.

7. Procédé sur la revendication 6, caractérisé en ce que la quantité d'acide désoxyribonucléique, dans la composition, est comprise entre 0,05 % et 2 % en poids de matière sèche de polymère.

8. Procédé sur l'une des revendications précédentes, caractérisé en ce que la cire est choisie parmi les cires animales, les cires minérales, les cires végétales, les cires synthétiques ou leur mélange.

9. Procédé selon l'une des revendications précédentes, caractérisé en ce que la composition comprend également un pigment choisi parmi les pigments minéraux, les pigments organiques, les pigments nacrés.

10. Procédé selon la revendication 9, caractérisé en ce que le pigment est compris dans la composition dans des proportions allant de 3 à 25 % en poids par rapport au poids total de la composition.

11. Procédé selon l'une des revendications précédentes, caractérisé en ce que la composition comprend également au moins un agent tensio-actif lorsqu'elle se présente sous forme d'une émulsion.

12. Procédé selon la revendication 11, caractérisé en ce que l'agent tensio-actif est présent dans la composition en une quantité allant de 2 à 30 % en poids par rapport au poids total de la composition.

13. Procédé selon l'une quelconque des revendications précédentes, caractérisé en ce que l'acide désoxyribonucléique est présent dans une phase aqueuse.

14. Procédé selon l'une quelconque des revendications précédentes, caractérisé en ce que la composition comprend au moins un agent épaississant d'origine naturelle ou synthétique.

15. Procédé selon l'une quelconque des revendications précédentes, caractérisé en ce que la composition comprend au moins un ingrédient choisi parmi les vitamines, les oligoléléments, les adoucissants, les conservateurs, les séquestrants, les parfums, les huiles, les silicones, les agents de cohésion, les polymères, les agents alcalinisants ou acidifiants, les charges.

16. Utilisation d'une composition telle que définie selon l'une quelconque des revendications 1 à 15 pour allonger et/ou recourber les cils et/ou les sourcils.

## Claims

1. Method of making up the eyes, characterized in that a composition comprising deoxyribonucleic acid or one of its inorganic or organic salts and at least one wax is applied to the lashes and/or the eyebrows.

2. Method according to Claim 1, characterized in that the composition comprises a cosmetically acceptable vehicle.

3. Method according to either of Claims 1 and 2, characterized in that the ratio by weight of the quantity of deoxyribonucleic acid to the quantity of wax is between 0.025 and 2.5.

4. Method according to one of the preceding claims, characterized in that the molecular weight of the deoxyribonucleic acid is between 1×10⁵ and 1×10⁷.

5. Method according to Claim 4, characterized in that the molecular weight of the deoxyribonucleic acid is between 5×10⁵ and 5×10⁶.

6. Method according to one of the preceding claims, characterized in that the quantity of deoxyribonucleic acid in the composition is between 0.02 % and 5 % by weight of dry polymer material.

7. Method according to Claim 6, characterized in that the quantity of deoxyribonucleic acid in the composition is between 0.05 % and 2 % by weight of dry polymer material.

8. Method according to one of the preceding claims, characterized in that the wax is chosen from animal waxes, mineral waxes, vegetable waxes and synthetic waxes or a mixture thereof.

9. Method according to one of the preceding claims, characterized in that the composition additionally comprises a pigment chosen from inorganic pigments, organic pigments and nacreous pigments.

10. Method according to Claim 9, characterized in that the pigment is present in the composition in proportions ranging from 3 to 25 % by weight relative to the total weight of the composition.

11. Method according to one of the preceding claims, characterized in that the composition additionally comprises at least one surfactant when it is presented in the form of an emulsion.

12. Method according to Claim 11, characterized in that the surfactant is present in the composition in a quantity ranging from 2 to 30 % by weight relative to the total weight of the composition.

13. Method according to any one of the preceding claims, characterized in that the deoxyribonucleic acid is present in an aqueous phase.

14. Method according to any one of the preceding claims, characterized in that the composition comprises at least one thickener of natural or synthetic origin.

15. Method according to any one of the preceding claims, characterized in that the composition comprises at least one ingredient chosen from vitamins, trace elements, emollients, preservatives, sequestering agents, fragrances, oils, silicones, cohesion agents, polymers, basifying or acidifying agents, and fillers.

16. Use of a composition as defined according to any one of Claims 1 to 15 for lengthening and/or curling the lashes and/or the eyebrows.

## Patentansprüche

1. Verfahren zum Schminken der Augen,
dadurch **gekennzeichnet,** daß
auf die Wimpern und/oder die Augenbrauen eine Zusammensetzung aufgetragen wird, die Desoxyribonucleinsäure oder ein anorganisches oder organisches Salz von Desoxyribonucleinsäure und mindestens ein Wachs enthält.

2. Verfahren nach Anspruch 1, dadurch **gekennzeichnet,** daß die Zusammensetzung einen kosmetisch akzeptablen Träger enthält.

3. Verfahren nach einem der Ansprüche 1 oder 2, dadurch **gekennzeichnet,** daß das Gewichtsverhältnis von Desoxyribonucleinsäure zu Wachs im Bereich von 0,025 bis 2,5 liegt.

4. Verfahren nach einem der vorhergehenden Ansprüche, dadurch **gekennzeichnet,** daß das Molekulargewicht der Desoxyribonucleinsäure im Bereich von 1·10⁵ bis 1·10⁷ liegt.

5. Verfahren nach Anspruch 5, dadurch **gekennzeichnet,** daß das Molekulargewicht der Desoxyribonucleinsäure im Bereich von 5·10⁵ bis 5·10⁶ liegt.

6. Verfahren nach einem der vorhergehenden Ansprüche, dadurch **gekennzeichnet,** daß der Mengenanteil der Desoxyribonucleinsäure in der Zusammensetzung im Bereich von 0,02 bis 5 Gew.-% Polymertrockensubstanz liegt.

7. Verfahren nach Anspruch 6, dadurch **gekennzeichnet,** daß der Mengenanteil der Desoxyribonucleinsäure in der Zusammensetzung im Bereich von 0,05 bis 2 Gew.-% Polymertrockensubstanz liegt.

8. Verfahren nach einem der vorhergehenden Ansprüche, dadurch **gekennzeichnet,** daß das Wachs unter den tierischen Wachsen, mineralischen Wachsen, pflanzlichen Wachsen, synthetischen Wachsen oder deren Gemischen ausgewählt ist.

9. Verfahren nach einem der vorhergehenden Ansprüche, dadurch **gekennzeichnet,** daß die Zusammensetzung ferner ein Pigment enthält, das unter den anorganischen Pigmenten, organischen Pigmenten und Perlglanzpigmenten ausgewählt ist.

10. Verfahren nach Anspruch 9, dadurch **gekennzeichnet,** daß das Pigment in der Zusammensetzung in Mengenanteilen von 3 bis 25 Gew.-%, bezogen auf das Gesamtgewicht der Zusammensetzung, vorliegt.

11. Verfahren nach einem der vorhergehenden Ansprüche, dadurch **gekennzeichnet,** daß die Zusammensetzung ferner mindestens ein grenzflächenaktives Mittel enthält, wenn sie in Form einer Emulsion vorliegt.

12. Verfahren nach Anspruch 11, dadurch **gekennzeichnet,** daß das grenzflächenaktive Mittel in der Zusammensetzung in einem Mengenanteil von 2 bis 30 Gew.-%, bezogen auf das Gesamtgewicht der Zusammensetzung, vorliegt.

13. Verfahren nach einem der vorhergehenden Ansprüche, dadurch **gekennzeichnet,** daß die Desoxyribonucleinsäure in einer wäßrigen Phase vorliegt.

14. Verfahren nach einem der vorhergehenden Ansprüche, dadurch **gekennzeichnet,** daß die Zusammensetzung mindestens ein Verdickungsmittel natürlichen oder synthetischen Ursprungs enthält.

15. Verfahren nach einem der vorhergehenden Ansprüche, dadurch **gekennzeichnet,** daß die Zusammensetzung mindestens einen Bestandteil enthält, der unter den Vitaminen, Spurenelementen, reizlindernden Mitteln, Konservierungsmitteln, Maskierungsmitteln, Parfums, Ölen, Siliconen, Haftmitteln, Polymeren, Alkalisierungs- oder Ansäuerungsmitteln und Füllstoffen ausgewählt ist.

16. Verwendung einer Zusammensetzung nach einem der Ansprüche 1 bis 15, um die Wimpern und/oder Augenbrauen zu verlängern und/oder zu krümmen.
